# EUROPEAN PATENT SPECIFICATION

(11) **EP 2 905 327 B1**
(45) Date of publication and mention of the grant of the patent: **04.07.2018**
(21) Application number: 14460131.7
(22) Date of filing: 23.12.2014
(51) Int. Cl.: C12M 1/107, C12M 1/00, C12M 1/12, C12M 1/26

(54) **SYSTEM FOR BIOGAS PRODUCTION UNDER ELEVATED PRESSURE**
SYSTEM ZUR BIOGASERZEUGUNG UNTER ERHÖHTEM DRUCK
SYSTÈME POUR LA PRODUCTION DE BIOGAZ SOUS PRESSION ÉLEVÉE

(30) Priority: 24.12.2013 PL 40670213
(43) Date of publication of application: 12.08.2015
(73) Proprietor: SYGMA Sp. z o.o., 54-211 Wroclaw (PL); Lukaszewicz, Marcin Ziemowit, 51-351 Wroclaw (PL); Sygit, Maciej, 54-211 Wroclaw (PL)
(72) Inventor: Lukaszewicz, Marcin Ziemowit, 51-351 Wroclaw (PL); Sygit, Maciej, 54-211 Wroclaw (PL)
(74) Representative: Winohradnik, J. Halina

(56) References cited:
- WO-A-86/05200
- DE-A1- 3 837 785
- DE-U1-202009 015 199
- GB-A- 2 131 786
- GB-A- 2 333 771

## Description

The subject matter of the invention is a system for biogas production under elevated pressure in a liquid anaerobic digestion process.

The systems used for anaerobic decomposition of materials containing organic carbon where the process leads to biogas generation, are provided with reactors for methane fermentation of organic materials by active anaerobic microorganisms.

Most often the process takes place in one large bioreactor under near-atmospheric gas pressure.

A self-contained bioreactor for anaerobic digestion of organic matter, comprising a horizontal vessel provided with an agitator and a compressor for compressing the biogas being returned to the bioreactor and used for mixing the reactants is known from the British patent specification GB 2131786 B.

A digestor feeding system known from the utility model DE 2009015199 comprises a substrate metering vessel provided with an agitator and a compressor, wherein the substrate being returned to the digestor undergoes break-up and is mixed with reactants that are put on digestion.

In the WO 86/05200 document described has been a method and a system comprising a gas tank and a compressor for gas compression, wherein the compressed biogas from the tank enters into the digestion chamber through a series of nozzles. The compressed biogas is employed in that system to mix and break-up the substrate, while the methanogenesis proceeds under atmospheric pressure.

The solution according to GB2333771 also provides for employment of compressed biogas to mix the substrate, and the biogas compression is achieved by means of energy from external sources.

A bioreactor filled with a bed to create a large contact surface area for reactants is known from the DE 3837785 document, where reactants as well as the substrate are recirculated in order to standardise reaction conditions.

A sewage treatment system comprising a pressure vessel for decomposition of organic material under elevated pressure is known from the WO 2011/005079A1 patent application under PCT procedure. The energy of compressed gas is utilised in this system for driving a pump involved in operation of the system. The patent application No. US 2007/0224669A1 presents a system, wherein the anaerobic digestion microorganisms are employed for pressure generation in a bioreactor and then for increasing methane content in biogas utilised by power industry. In other system, known from a description in the application No. WO 2008/142007 under PCT procedure, a digester is placed in front of a single-vessel reactor and packed with tubes that afford possibilities for formation of a biofilm consisting of microorganisms, owing to which hydrolytic reactions have been partially separated from the actual methanogenesis, moreover, the system is provided with a sedimentation chamber.

The essence of the present invention constitutes a system for processing organic matter by anaerobic digestion under spontaneously generated elevated pressure.

The system comprises a substrate tank connected to a pneumatic substrate metering vessel, the top part of which, by a biogas receiving pipeline provided with a gas valve, is connected to a low-pressure gas tank, where the tank pressure is preferably close to the atmospheric pressure, and by a gas supplying pipeline provided with a valve, to a high-pressure gas tank, where the gas pressure is preferably above 8 bars. Furthermore, the bottom head of the pneumatic metering vessel is connected to a bioreactor by a pipeline provided with a valve.
The bioreactor is filled with a high-surface area bed for development of a biofilm acting as a catalyst in biochemical reactions. A separatory zone for the biogas separation is situated in the upper part of the bioreactor, and is connected by a pipeline provided with a digestate valve to a digestate tank, into which the digestate from the bioreactor separatory zone is carried away. A gas pipeline provided with a valve and connected to the upper wall of the bioreactor takes the biogas off the bioreactor to a high-pressure gas tank. To the same upper wall of the bioreactor connected is also another pipeline provided with a gas valve, to take off the biogas to a low-pressure gas tank. The sedimenting fraction is carried away to the digestate tank by a discharge pipeline provided with a valve and outgoing from the bottom of the bioreactor. Preferably, the gas valves, digestate valve and the valve that receives the sedimenting fraction to the digestate tank, are computer controlled. Preferably, the system is provided with pressure gauges, and the bioreactor is provided with temperature and pH sensors as well as with a temperature control system.

The system enables operating the process under elevated pulsating pressure, which makes possible a reciprocating movement of the substrate and mixing the reaction substrate without using mechanical agitators.

The system is characteristic of its design, which enables to utilise the pneumatic energy, self-generated during processing the organic matter, for operation of the system, without need to use external energy sources. The pneumatic energy is stored in the high-pressure vessel in the similar way as it occurs in a gas tank of a compressor, and the pneumatic substrate metering vessel substitutes the operation of a mechanical metering pump, and thus makes possible to perform anaerobic digestion process in the bioreactor under pulsating pressure.

The system according to the invention but unprovided with pumps and agitators, although of rather unsophisticated design and relatively small in size, while under constant pressure supervision, it may enable acceleration of the anaerobic digestion and optimal conditions for biochemical reactions carried out therein.

The important advantage is higher methane content over CO₂ in the biogas obtained in the system, and the reason is that CO₂ for the most part dissolves in the liquid phase and, owing to elevated pressure, is carried away to the digestate tank.

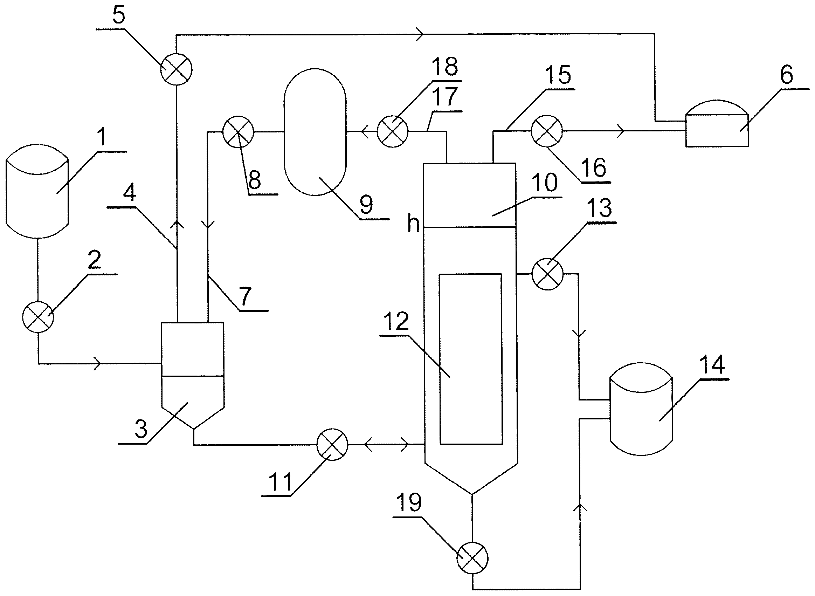

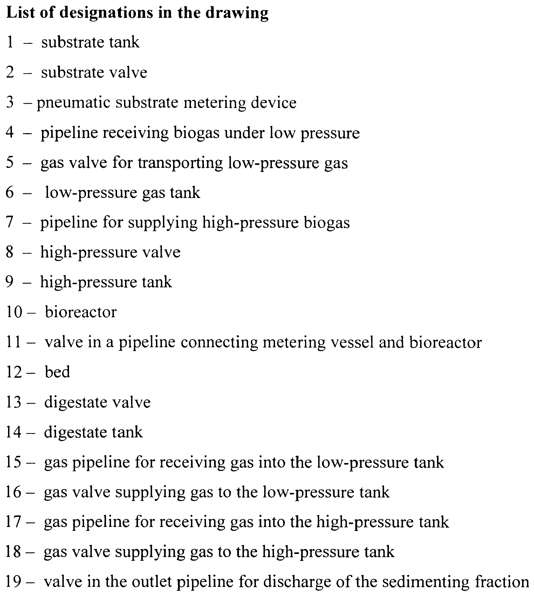

The subject matter of the invention has been explained in the embodiment and in the drawing.

### Embodiment

The system for biogas production consists of a substrate tank **1**, connected through a valve **2** to a pneumatic substrate metering vessel **3**, in the lid of which mounted is a pipeline **4** to carry away the biogas, provided with a computer controlled gas valve **5**, and leading to a low-pressure gas tank **6**, where the pressure is of near-atmospheric level. Furthermore, in the lid of the pneumatic substrate metering vessel **3** mounted is a biogas supplying pipeline **7**, with the biogas being fed under elevted pressure through a computer controlled gas valve **8**, provided in the said supplying pipeline, from a high-pressure gas tank **9**, where the gas pressure is 8 bar. In addition, the bottom head of the substrate metering vessel **3** is connected to the bioreactor **10** through a valve **11.** A bed **12** made of a rolled board of a horizontal section in shape of Archimedes' spiral is packed in the bioreactor **10**. In the upper part of the bioreactor **10** located is a separatory zone where separation of the biogas takes place, and this zone is connected to a digestate tank **14** by a pipeline provided with a digestate valve **13.** The separated biogas under near-atmospheric pressure is received from the separatory zone of the bioreactor to a gas pipeline **15** provided with a gas valve **16**, embedded in the upper wall of the bioreactor **10**, the said pipeline transports the gas to the gas tank **6** under near-atmospheric pressure. The separated biogas under elevated pressure is supplied to a high-pressure gas tank **9** by a gas pipeline **17** connected to the upper wall of the bioreactor and provided with a gas valve **18.** The sedimenting fraction is discharged through an outlet pipeline mounted in the bottom head of the bioreactor and provided with a valve **19**, to the digestate tank **14**. A pressure gauge is located in the upper part of the bioreactor **10.** A pressure gauge is placed also in the high-pressure tank **9.** Furthermore, the bioreactor is provided with temperature and pH sensors, and also with temperature control system.

### Operation of the system according to the invention:

In order to start up the system, the high-pressure gas tank **9** to be filled with methane and the substrate is loaded through the substrate valve **2** into the pneumatic metering vessel **3** while the valve **5** in the low-pressure biogas receiving pipeline **4** remains open. Once the pneumatic vessel **3** is full, both computer-controlled valves get automatically closed, and after the valve **8** in the pipeline **7**, that supplies gas under elevated pressure, and the valve **11** have been automatically opened, the substrate is fed into the bioreactor **10**, where the anaerobic microbiological digestion process takes place. The produced biogas under the pressure not lower than the pressure in the high-pressure tank, having been separated in the separatory zone of the bioreactor **10**, is received into the high-pressure tank **9.** In case the reaction mixture table level exceeds the set value **h**, the computer-controlled valve **13** automatically opens and a part of digestate is removed to the digestate tank **14** in quantity sufficient to lower the reaction mixture table level to the set level **h.** In order to replenish the bioreactor with the substrate, the digestate valve **13** gets automatically closed and, at open valve **11** and valve **8** in the pipeline **7** which supplies gas under pressure to the pneumatic vessel **3**, the substrate is fed into the bioreactor **10.** At the same time, in order to intensify agitation of the contents in the bioreactor **10**, the valve **15**, which removes the biogas to the low-pressure tank 6, automatically opens in a pulsing mode.

In order to replenish the contents of the pneumatic substrate metering vessel **3**, upon closing the valve **11**, which delivers the substrate to the bioreactor **10**, and closing the valve **8**, which delivers compressed gas to the pneumatic vessel **3**, now the valve **4** opens, removing the biogas to the low-pressure tank **6**, and the pressure drop in the pneumatic vessel **3** effects in opening the valve **2**, so the substrate is delivered to the pneumatic vessel **3**, forcing out the remaining biogas into the low-pressure tank at the same time. Insoluble or hardly soluble components collecting in the bottom part of the bioreactor **10** are transferred to the digestate tank **14** once the valve **19** has been opened.

Instead of using an agitator in the system, in order to improve blending of the reaction mixture and to provide its reciprocating movement, the following actions are executed automatically: opening the valve **11** results in return of the reaction mixture from the bioreactor **10** to the pneumatic vessel **3** and compression of the gas cushion above the substrate followed by opening the valve **8,** owing to which the pneumatic energy accumulated in the high-pressure tank **9** forces the substrate to be pushed out in reciprocating moves to the bioreactor **10**. Periodical opening the valve **19**, discharging the sedimenting fraction, causes that the reaction mixture in the bioreactor **10** undergoes blending.

## Claims

1. A system for production of biogas under elevated pressure by subjecting organic matter to anaerobic digestion, provided with a bioreactor **characterised in that** the said system operates under spontaneously generated elevated pressure and comprises a substrate tank (1), connected to a pneumatic substrate metering vessel (3) the upper part of which by a biogas receiving pipeline (4) is connected to a low-pressure gas tank (6) through a gas valve (5), and by a gas supplying pipeline (7) provided with a valve (8) to a high-pressure gas tank (9), furthermore a bottom head of the said pneumatic metering vessel (3) is connected by a pipeline provided with a valve (11) to the said bioreactor (10), in the upper part of which located is a separatory zone for the biogas separation, where the said zone is connected by a pipeline provided with a digestate valve (13) to a digestate tank (14), to the upper wall of the said bioreactor (10) connected are: a gas pipeline (17) provided with a valve (18), for receiving biogas into the said high-pressure gas tank (9), and a pipeline (15) provided with a gas valve (16), for removing the biogas to the said low-pressure gas tank (6), in addition, an outlet pipeline provided with a valve (19) is mounted in the bottom head of the said bioreactor (10) for discharging the sedimenting fraction into the digestate tank (14).

2. A system according to claim 1, wherein the said high-pressure gas tank (9) operates under the pressure higher than 8 bar, while the said low-pressure gas tank (6) - under the near-atmospheric pressure.

3. A system according to claim 1, wherein the said bioreactor (10) is packed with the said large-surface bed (12) for development of a biofilm catalysing biochemical reactions.

4. A system according to claim 1, wherein all said valves are computer-controlled.

5. A system according to claim 1, **characterised in that** the said system is provided with pressure, temperature and pH sensors, as well as a temperature control system.

## Patentansprüche

1. System zur Biogaserzeugung unter erhöhtem Druck durch Verarbeitung von organischem Material im Prozess der anaeroben Fermentation mit einem Bioreaktor, **dadurch gekennzeichnet, dass** das System unter spontan erzeugtem erhöhtem Druck arbeitet und einen Substrattank (1) umfasst, der mit einem pneumatischen Dosierbehälter (3) verbunden ist, dessen oberer Teil durch eine Biogas-Aufnahme-Rohrleitung (4) mit einem Niederdruck-Gasbehälter (6) durch ein Gasventil (5) und durch eine mit einer Gaszuführungsrohrleitung (7) versehene Leitung verbunden ist, versorgt mit Ventil (8) zu einem Hochdruckgasbehälter (9), wobei weiterhin ein Bodenkopf des pneumatischen Dosierbehälters (3) durch eine mit einem Ventil (11) versehene Rohrleitung mit dem Bioreaktor (10) verbunden ist oberer Teil davon ist eine Trennzone für die Biogasabtrennung, wobei die Zone durch eine mit einem Gärrestventil (13) versehene Leitung mit einem Gärrestbehälter (14) mit der oberen Wand des Bioreaktors (10) verbunden ist verbunden sind: eine Gasleitung (17), die mit einem Ventil (18) zur Aufnahme von Biogas in den genannten Hochdruckgastank (9) und einer mit einem Gasventil (16) versehenen Rohrleitung (15) zum Abführen des Biogases in den Niederdruckgastank (6), zusätzlich gibt es eine Auslassleitung, die mit einem Ventil (19) versehen ist, in dem Boden des Bioreaktors (10) angebracht, um die sedimentierende Fraktion in den Gärresttank (14) abzuführen.

2. System gemäß Anspruch 1, **dadurch gekennzeichnet, dass** Hochdruckgastank (9) unter dem Druck höher als 8 bar arbeitet, während der Niederdruckgastank (6) unter dem nahezu atmosphärischen Druck arbeitet.

3. System gemäß Anspruch 1, **dadurch gekennzeichnet, dass** der Bioreaktor (10) mit dem Großflächenbett (12) zur Entwicklung eines biochemischen Reaktionen katalysierenden Biofilms gepackt ist.

4. System gemäß Anspruch 1, **dadurch gekennzeichnet, dass** alle Ventile computergesteuert sind.

5. System gemäß Anspruch 1, **dadurch gekennzeichnet, dass** das System mit Druck-, Temperatur- und pH-Sensoren sowie einem Temperaturkontrollsystem versehen ist.

## Revendications

1. Système pour la production de biogaz sous pression élevée par traitement de matières organiques fermentées en milieu anaérobie, équipé d'un bioréacteur, **caractérisé en ce qu'**il fonctionne par pression générée spontanément ; il comprend un réservoir de substrat (1) relié à un réservoir pneumatique de dosage de substrat (3) dont la partie supérieure est raccordée par une conduite de biogaz (4) à un réservoir de gaz à basse pression (6) via une vanne de gaz (5) et reçoit une conduite acheminant du gaz (7) via une vanne (8) depuis un réservoir de gaz à haute pression (9) ; le fond du réservoir pneumatique de dosage (3) est raccordé par une conduite équipée d'une vanne (11) à un bioréacteur (10) comportant dans sa partie supérieure une zone de séparation dans laquelle le biogaz est recueilli ; cette zone de séparation est raccordée par une conduite munie d'une vanne d'évacuation (13) à un réservoir de digestats (14) ; de la paroi supérieure du bioréacteur (10), partent une conduite de gaz (17) avec vanne (18) destinée à acheminer du biogaz vers le réservoir de gaz à haute pression (9), et une conduite (15) équipée d'une vanne (16) pour acheminer du biogaz vers le réservoir de gaz à basse pression (6) ; en outre, le fond du bioréacteur (10) est pourvu d'une conduite munie d'une vanne (19) servant à évacuer la fraction sédimentaire vers le réservoir de digestats (14).

2. Système selon la revendication 1, **caractérisé en ce que** le réservoir de gaz à haute pression (9) fonctionne sous une pression supérieure à 8 bars, et le réservoir de gaz à basse pression (6) a un niveau proche de la pression atmosphérique.

3. Système selon la revendication 1, **caractérisé en ce que** le bioréacteur (12) est rempli d'un lit de substrat de grande surface destiné au développement du biofilm catalyseur de réactions biochimiques.

4. Système selon la revendication 1, **caractérisé en ce que** toutes les vannes sont contrôlées par ordinateur.

5. Système selon la revendication 1, **caractérisé en ce qu'**il est équipé de capteurs de pression, de température et de pH et d'un système de régulation de température.
